# EUROPEAN PATENT APPLICATION

(11) **EP 4 498 083 A1**
(43) Date of publication of application: **29.01.2025**
(21) Application number: 23774196.2
(22) Date of filing: 13.01.2023
(51) Int. Cl.: G01N 33/14

(54) **TASTE INFORMATION PROVIDING METHOD AND PROGRAM**

(30) Priority: 22.03.2022 JP 2022045103
(71) Applicant: SHIMADZU CORPORATION, Kyoto-shi, Kyoto 604-8511 (JP)
(72) Inventor: MATSUMOTO, Keiko, Kyoto-shi, Kyoto 604-8511 (JP)
(74) Representative: Müller-Boré & Partner Patentanwälte PartG mbB
(86) International application number: PCT/JP2023/000829
(87) International publication number: WO 2023/181595

(57) **Abstract**

Provided is a technique for providing objective and highly accurate information for taste evaluation. A device acquires information about respective amounts of two or more types of components in a target (step S21). The two or more types of components are associated with a specific taste. The device derives a determination result as to whether the target has the specific taste, based on ratio information about a ratio of each of the two or more types of components and information about an amount of each of the two or more types of components (step S23), and outputs the determination result (step S24).

## Description

### TECHNICAL FIELD

The present invention relates to a taste information providing method, and particularly relates to providing information about a specific taste of a target.

### BACKGROUND ART

Various characteristics of tastes have been defined for various foods, and various studies are being conducted for providing information about each taste. For example, the tastes having been defined for sake (a Japanese alcoholic beverage) include the one called "blossoming taste" ("fukurami" in Japanese). The term "blossoming taste" is widely and generally used by those who enjoy sake for expressing a flavor of sake. While "blossoming taste" has no explicit definition, NPL 1, for example, defines it as "a flavor and an aroma spread roundly in the mouth" and "a flavor and a similar aroma rise into the nose and linger long."

Evaluation of a specific taste of a food depends largely on evaluation by sensory analysis assessors. Under such a circumstance, many attempts are being made to evaluate various tastes by making use of the results of component analysis. For example, NPL 2 reports an evaluation based on the results of analysis of respective contents of specific components, to determine whether each type of sake has the above-identified "blossoming taste" (reports that those types of sake having low contents of ethanolamine, glycine, and proline have "blossoming taste") (see NPL 2, for example).

### CITATION LIST

### NON PATENT LITERATURE

NPL 1: Nihon-Shu Ajiwai Jiten (Glossary of Sake Tasting), blossoming taste, online, August 29, 2019 (searched for on March 8, 2022), Internet <URL:https://twitter.com/sakedic/status/1167044555163623425>
NPL 2: Kimio IWANO, Toshihiko ITO, and Nobushige NAKAZAWA "Correlation Analysis of a Sensory Evaluation and the Chemical Components of Ginjyo-shu," Journal of the Brewing Society of Japan, Japan, the Brewing Society of Japan, September 15, 2005, Vol. 100, No. 9, pp. 639-649

### SUMMARY OF INVENTION

### TECHNICAL PROBLEM

Evaluation that makes use of component analysis is expected to provide objective information about tastes. Thus, there is a need for a system for providing information of higher accuracy as such objective information.

The present invention has been devised in view of the above circumstances, and an object thereof is to provide a technique for providing objective and highly accurate information for taste evaluation.

### SOLUTION TO PROBLEM

A taste information providing method according to an aspect of the present disclosure is a method for providing taste information about a specific taste, and the method includes: acquiring target ratio information about respective ratios of two or more types of components in a target, the two or more types of components being associated with the specific taste; deriving a determination result for the specific taste of the target, based on the target ratio information and criterion ratio information about respective ratios of the two or more types of components; and outputting the determination result.

A taste information providing method according to another aspect of the present disclosure is a method for providing taste information about a specific taste, and the method includes: acquiring information about an amount of each component of two or more types of components in a target, the two or more types of components being associated with the specific taste; calculating a ratio of the amount of each component to a sum of respective amounts of the two or more types of components, based on the information about the amount of each component of the two or more types of components; and outputting the ratio of the amount of each component.

A program according to an aspect of the present disclosure is a program for providing taste information, the program being executed by a processor of a computer to cause the computer to perform the taste information providing method as described above.

### ADVANTAGEOUS EFFECTS OF INVENTION

According to the present disclosure, objective and highly accurate information is provided for taste evaluation.

### BRIEF DESCRIPTION OF DRAWINGS

Fig. 1 is a block diagram illustrating a configuration of a device 100 for providing taste information.
Fig. 2 is a diagram illustrating information about eight types of sake used for selecting marker components.
Fig. 3 is a diagram illustrating analytical conditions for a high-performance liquid chromatograph (HPLC) and a mass spectrometer (MS) for identifying flavor components.
Fig. 4 is a diagram illustrating a score plot produced as a result of principal component analysis on the results of identification of flavor components.
Fig. 5 is a diagram illustrating a loading plot produced as a result of principal component analysis on the results of identification of flavor components.
Fig. 6 is a diagram illustrating a score plot produced as a result of partial least squares-discriminant analysis on the results of identification of flavor components.
Fig. 7 is a diagram illustrating a loading plot produced as a result of partial least squares-discriminant analysis on the results of identification of flavor components.
Fig. 8 is a diagram illustrating test statistics of the partial least squares-discriminant analysis described with reference to Figs. 6 and 7.
Fig. 9 is a diagram illustrating a difference in the value of the area between a sample having blossoming taste and a sample having no blossoming taste, for four types of components among 10 types of components shown in Fig. 8.
Fig. 10 is a diagram illustrating one example of a screen to be provided as information about blossoming taste.
Fig. 11 is a flowchart of one example of a process performed by device 100 to provide information about taste.
Fig. 12 is a diagram illustrating a screen that is displayed in the modification of step S14.
Fig. 13 is a flowchart of another example of the process performed by device 100 to provide information about taste.
Fig. 14 is a diagram illustrating a specific example of respective peak areas of marker components and a determination result, for two types of targets.
Fig. 15 is a diagram illustrating one example of a screen for displaying a determination result.
Fig. 16 is a diagram illustrating analytical conditions for a gas chromatograph (GC) and a mass spectrometer (MS) for identifying aroma components.
Fig. 17 is a diagram illustrating a score plot produced as a result of principal component analysis on the results of identification of aroma components.
Fig. 18 is a diagram illustrating a loading plot produced as a result of principal component analysis on the results of identification of aroma components.
Fig. 19 is a diagram illustrating a score plot produced as a result of partial least squares-discriminant analysis on the results of identification of aroma components.
Fig. 20 is a diagram illustrating a loading plot produced as a result of partial least squares-discriminant analysis on the results of identification of aroma components.
Fig. 21 is a diagram illustrating test statistics of the partial least squares-discriminant analysis described with reference to Figs. 19 and 20.
Fig. 22 is a diagram illustrating a difference in area value between samples having blossoming taste and samples having no blossoming taste, for two types of components shown in Fig. 21.
Fig. 23 is a diagram illustrating a specific example of respective peak areas of marker components and a determination result, for two types of targets.
Fig. 24 is a block diagram illustrating a configuration of a device 100A for providing taste information.
Fig. 25 is a flowchart of one example of a process performed by device 100A.
Fig. 26 is a flowchart of a process performed to provide information about taste in Embodiment 4.

### DESCRIPTION OF EMBODIMENTS

Embodiments of the present disclosure are described in detail hereinafter with reference to the drawings. In the drawings, the same or corresponding parts are denoted by the same reference characters, and a description thereof is not herein repeated.

### [Embodiment 1]

### <Configuration of Device>

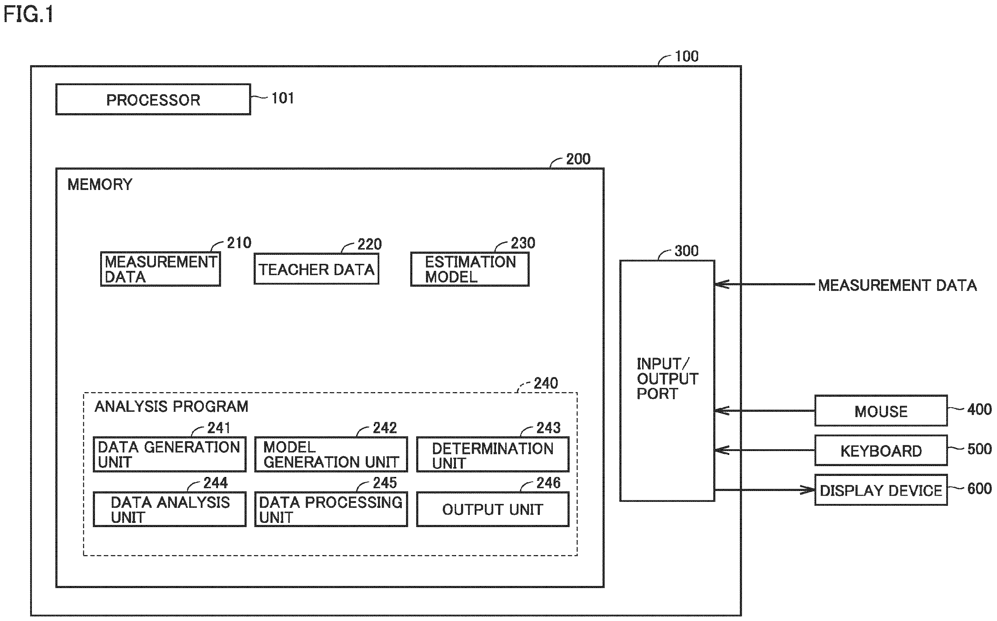

Fig. 1 is a block diagram illustrating a configuration of a device 100 for providing taste information. One example of the taste information to be provided is the ratio of each of a plurality of different types of components selected for a specific taste. Another example thereof is a result of determination as to whether a specific taste is present or not.

Device 100 is configured on the basis of a personal computer, for example. Device 100 may also be configured in the form of a server accessible from one or more terminal devices through a network such as the Internet.

Device 100 includes a processor 101, a memory 200, and an input/output port 300. A mouse 400, a keyboard 500, and a display device 600 are connected to input/output port 300. An analyzer such as chromatograph may be connected to input/output port 300. One or more terminal devices may also be connected to input/output port 300 through the Internet, a local area network, or the like. Device 100 itself may include at least some of mouse 400, keyboard 500, and display device 600.

Measurement data is input to input/output port 300. The measurement data is used in some cases for providing the taste information, and used in some cases for producing teacher data for an estimation model.

Memory 200 stores measurement data 210, teacher data 220, an estimation model 230, and an analysis program 240 for performing an analysis process and a machine learning process. They may be stored in memory 200 non-temporarily. In one implementation, the measurement data may be input to device 100 through a chromatograph connected to input/output port 300.

Teacher data 220 includes a data set prepared for each of two or more samples. Each data set includes a peak area of each of given types of components identified from measurement data of the sample, and a determination result applied to the sample. The determination result represents a result of determination, made by a sensory analysis assessor, as to whether or not each sample has a specific taste. The given type of component is described later herein as a marker component.

Estimation model 230 is a program for performing calculation in accordance with the model. In one implementation, estimation model 230 is implemented as a discriminant model generated by statistical analysis software (for example, eMSTAT (registered trademark): https://www.an.shimadzu.co.jp/ms/emstat/index.htm manufactured by Shimadzu Corporation), and conforms to an algorithm "Support Vector Machine." It should be noted that the algorithm of estimation model 230 is not limited to this but may be any algorithm capable of determining whether a target has a specific taste or not, based on input data relating to the target (the input data is, for example, a value relating to the amount of each of given types of components of the target).

Analysis program 240 includes, as its functions, a data generation unit 241, a model generation unit 242, a determination unit 243, a data analysis unit 244, a data processing unit 245, and an output unit 246. In one implementation, respective functions of data generation unit 241, model generation unit 242, determination unit 243, data analysis unit 244, data processing unit 245, and output unit 246 are implemented through execution of a given program by processor 101.

Data generation unit 241 generates teacher data 220 from measurement data 210. More specifically, for each sample, data generation unit 241 identifies a peak area value, a peak height, or a component concentration of each of given types of components, based on measurement data 210, and applies the above-described determination result to the peak area to generate teacher data 220 for each sample. The peak picking technique may be used for identifying the peak area.

Model generation unit 242 uses teacher data 220 to perform a machine learning process on estimation model 230. Estimation model 230 having been subjected to the machine learning process is also referred to as "learned model" herein.

Determination unit 243 applies the measurement data of a target to the learned model to thereby derive a determination result for the target.

Data analysis unit 244 uses measurement data 210 to perform a process for selecting the given types of components from components contained in a sample. The selection of the given types of components is described later herein as selection of marker components.

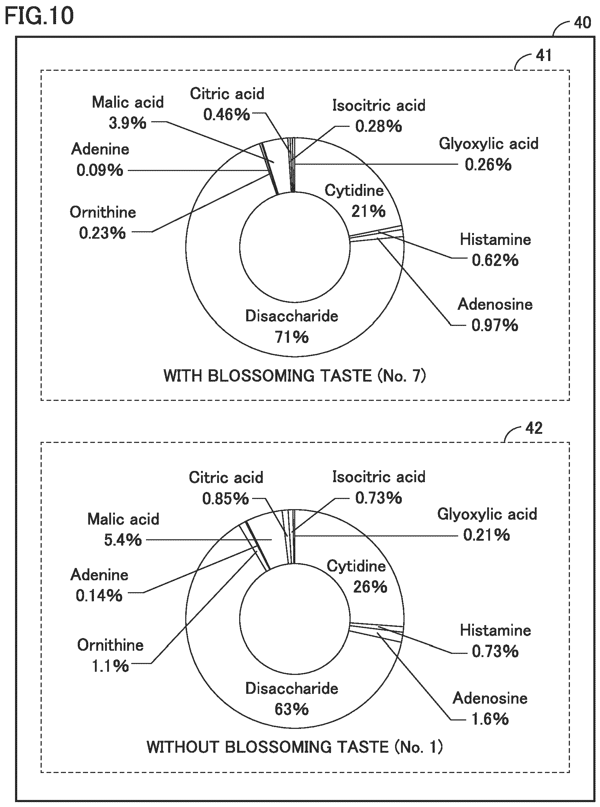

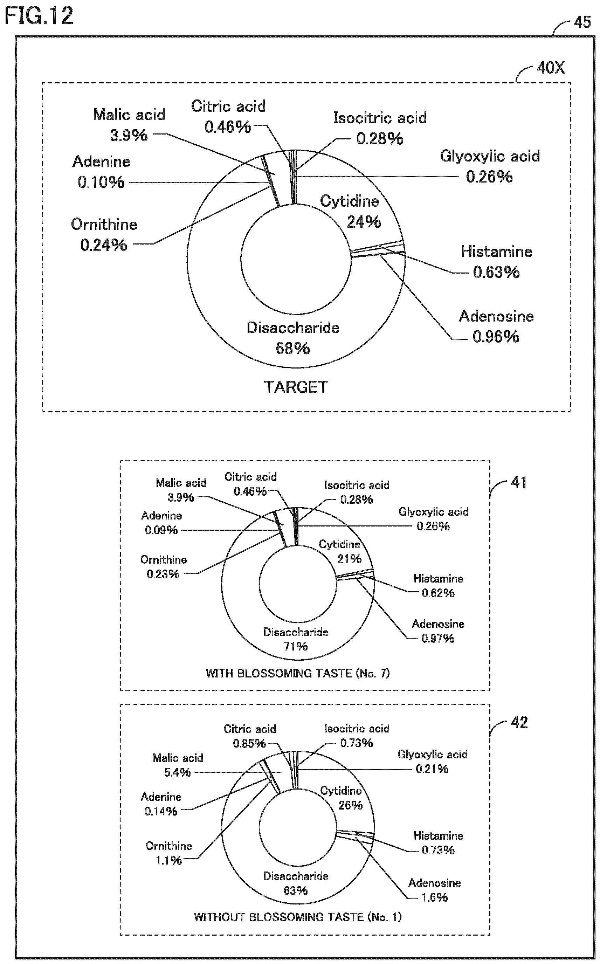

Data processing unit 245 processes measurement data 210 to thereby generate graphs as described with reference to Figs. 10 and 12.

Output unit 246 generates image information for displaying the determination result derived by determination unit 243 as well as the graphs generated by data processing unit 245, and instructs display device 600 (or an external device) to display the image information.

### <Selection of Marker Components>

As described above, in the present embodiment, marker components are used to provide information about a specific taste. In the following, a specific example of selection of marker components is described. In the following example, "blossoming taste" of sake is adopted as one example of specific tastes.

### (1) Used types of sake

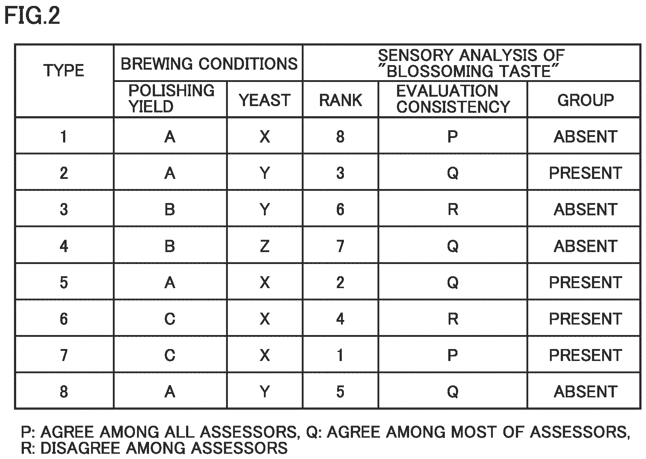

Fig. 2 is a diagram illustrating information about eight types of sake used for selecting marker components. In Fig. 2, "No. 1" to "No. 8" are indicated as information for identifying the eight types of sake, respectively.

Fig. 2 further illustrates brewing conditions and sensory analysis of "blossoming taste" for each type of sake. The brewing conditions include two items (polishing yield and yeast). The polishing yield represents a proportion of rice used for brewing that remains after being polished, and includes three values (A, B and C). Yeast represents a type of yeast used for brewing, and includes three values (X, Y and Z).

The sensory analysis of "blossoming taste" includes three items (rank, evaluation consistency, and group). The rank represents a rank assigned by a plurality of assessors. The rank for the first place indicates that the type of sake granted the first place has the maximum degree of "blossoming taste" among the eight types of sake. The evaluation consistency represents the degree of agreement in evaluation among a plurality of assessors, and includes three values (P, Q, and R). "P" indicates that a plurality of assessors give the same rating. "Q" indicates that most of a plurality of assessors give the same rating. "R" indicates that a plurality of assessors give different ratings. Group represents a group into which each type of sake is classified according to the rank granted to the type of sake, and includes two values (present, and, absent). "Present" represents a group having "blossoming taste." "Absent" represents a group having no "blossoming taste." In the example of Fig. 2, Nos. 2 and 5 to 7 belong to the group having "blossoming taste" and Nos. 1, 3, 4, and 8 belong to the group having no "blossoming taste." It is seen from Fig. 2 that some types of sake have "R" as a value representing evaluation consistency, and thus it is difficult in some cases for assessors to evaluate "blossoming taste."

### (2) Identification of flavor components

In order to obtain analysis data for selecting marker components, three samples were produced from each of the eight types of sake. That is, 24 samples were produced in total. In each sample, sake was diluted 10-fold with water.

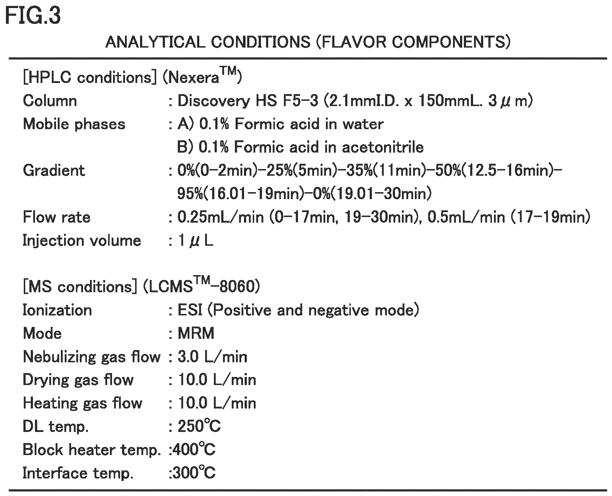

Fig. 3 is a diagram illustrating analytical conditions for a high-performance liquid chromatograph (HPLC) and a mass spectrometer (MS) for identifying flavor components. The ion pair-free LC/MS/MS method included in the "LC/MS/MS Method Package for Primary Metabolites Ver. 3" (https://www.an.shimadzu.co.jp/lcms/tq-option/mp_primary-metabolites.htm) was used for each of the 24 samples, and 153 types of components (flavor components) set by adding saccharide to hydrophilic metabolites such as amino acids, organic acids, nucleosides, and nucleotides, which are important in metabolome analysis in the life science field, were identified in accordance with the conditions indicated in Fig. 3.

The results of the above identification are input as measurement data to device 100 via input/output port 300.

### (3) Data analysis by principal component analysis (PCA)

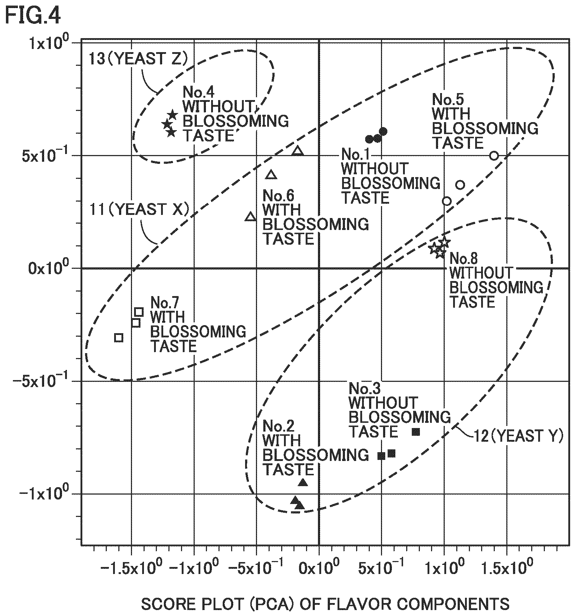

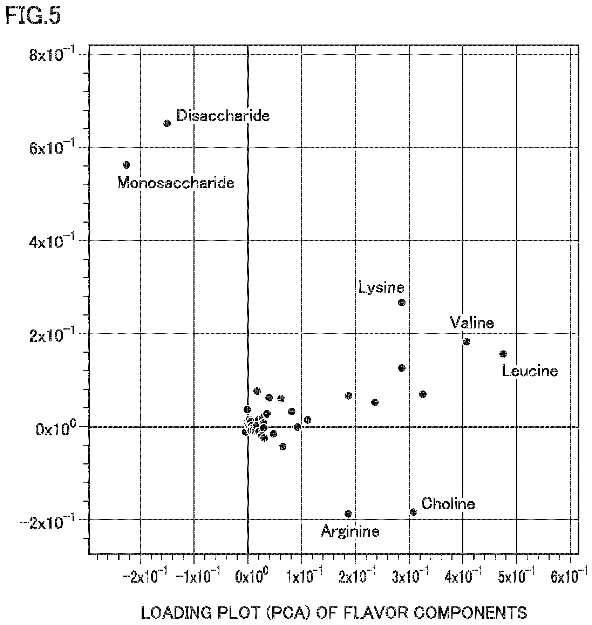

Fig. 4 is a diagram illustrating a score plot produced as a result of principal component analysis on the results of identification of flavor components. Fig. 5 is a diagram illustrating a loading plot produced as a result of principal component analysis on the results of identification of flavor components.

The score plot shown in Fig. 4 and the loading plot shown in Fig. 5 illustrate the results of the principal component analysis by statistical analysis software (for example, eMSTAT (registered trademark): https://www.an.shimadzu.co.jp/ms/emstat/index.htm manufactured by Shimadzu Corporation), using the area ratio (internal standard: 2-Morpholinoethanesulfonic acid) of each of the flavor components identified as described above.

As illustrated in Fig. 4, for each of No. 1 to No. 8, respective points representing the three samples produced from the same type of sake are located at positions close to each other in the score plot. In Fig. 4, in the vicinity of the points of each of No. 1 to No. 8, a value of a group ("group" in Fig. 2) to which each type belongs, that is, "blossoming taste is present" or "blossoming taste is absent" is added. For example, Fig. 2 indicates that the sake of No. 1 belongs to the group: blossoming taste is "absent." In Fig. 4, the indication "blossoming taste is absent" is added in the vicinity of the three points for No. 1.

In Fig. 4, regions 11, 12, and 13 each represent a type of yeast (see Fig. 2) used for brewing each type of sake. Region 11 represents a region in which points corresponding to the types of sake (Nos. 1 and 5 to 7) for which yeast X is used are located. Region 12 represents a region in which points corresponding to the types of sake (Nos. 2, 3, and 8) for which yeast Y is used are located. Region 13 represents a region in which points corresponding to the type of sake (No. 4) for which yeast Z is used are located. According to the results illustrated in Fig. 4, sake may be classified by the type of yeast used for sake.

In Fig. 5, the names of compounds of principal components are added. The added names of compounds include Disaccharide, Monosaccharide, Lysine, Valine, Leucine, Choline, and Arginine.

It is seen from Figs. 4 and 5 that sake may be classified by the type of yeast used for sake, based on principal component analysis. The second quadrant is characterized by monosaccharide and disaccharide, and the first quadrant and the fourth quadrant are characterized by amino acids such as leucine and valine. However, in Fig. 4, region 11 is located between region 12 and region 13. It is therefore difficult to derive a condition(s) for distinguishing the region where the points "with blossoming taste" are located, from the region where the points "without blossoming taste" are located on the score plot.

Thus, as seen from the results illustrated in Fig. 4 and Fig. 5, it is difficult to find a component(s) that determine whether or not sake has "blossoming taste" based on principal component analysis.

### (4) Data analysis by partial least squares-discriminant analysis (PLS-DA)

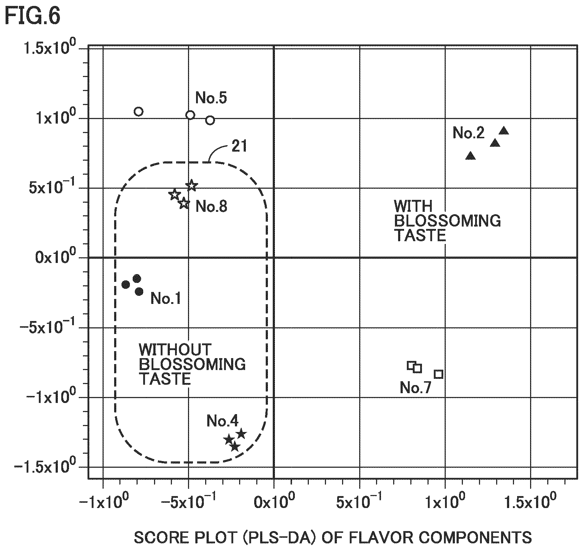

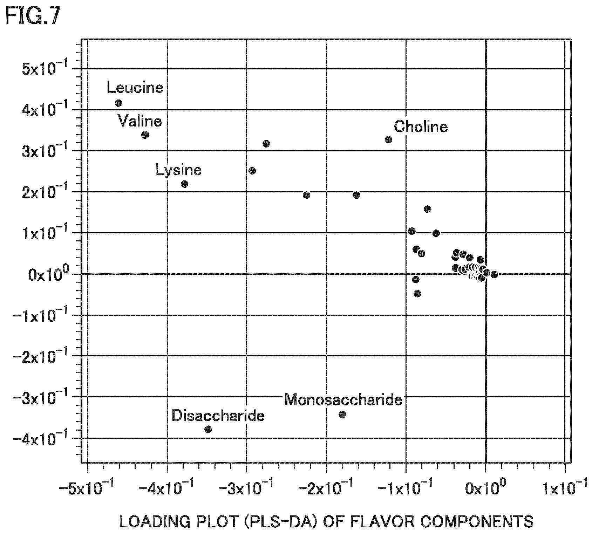

Fig. 6 is a diagram illustrating a score plot produced as a result of partial least squares-discriminant analysis on the results of identification of flavor components. Fig. 7 is a diagram illustrating a loading plot produced as a result of partial least squares-discriminant analysis on the results of identification of flavor components. In Fig. 6 and Fig. 7, the types of sake for which a plurality of assessors disagree about evaluation (No. 3 and No. 6 to which the value "R" of "evaluation consistency" is assigned in Fig. 2) are not analyzed.

In the score plot illustrated in Fig. 6, the points for the types of sake "without blossoming taste" (Nos. 1, 4, and 8) are located in the second quadrant and the third quadrant. Specifically, first principal components of these types of sake form a group in the negative region in the score plot. In the loading plot, points of amino acid and saccharide compounds (leucine, valine, lysine, and disaccharide) are located in the region corresponding to the negative region in the score plot. Thus, it is seen that these compounds are contained at a high content in the types of sake evaluated as the one "without blossoming taste."

### (5) Marker components

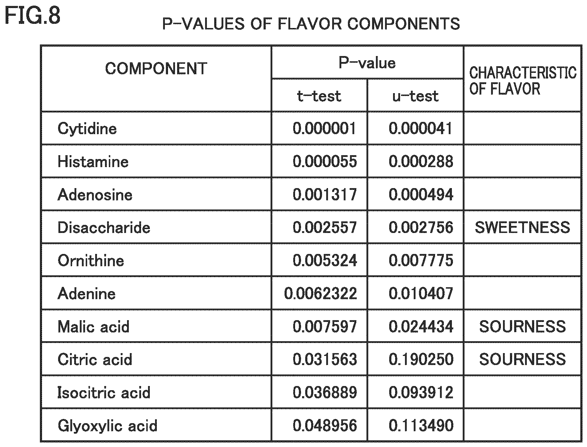

Fig. 8 is a diagram illustrating test statistics of the partial least squares-discriminant analysis described with reference to Figs. 6 and 7. Fig. 8 illustrates respective test statistics (p-values) for 10 types of components (Cytidine, Histamine, Adenosine, Disaccharide, Ornithine, Adenine, Malic acid, Citric acid, Isocitric acid, and Glyoxylic acid). These 10 types of components are contained in No. 1 to No. 8.

In Fig. 8, statistics for t-test and statistics for u-test are illustrated as test statistics. The statistics for t-test and the statistics for u-test are examples of test statistics calculated to analyze the difference between the type of sake having "blossoming taste" and the type of sake having no "blossoming taste."

It is considered that a component having a low test statistic is a component of which content is different to a relatively large degree between the sake group having "blossoming taste" and the sake group having no "blossoming taste." In the present embodiment, 10 types of components shown in Fig. 8 are selected as such components (marker components). The criterion for this selection is that respective values of the statistic for the t-test and the statistic for the u-test are both 0.05 or less. Device 100 stores, in memory 200, information for identifying the selected components in association with "blossoming taste."

The 10 types shown in Fig. 8 include many organic acids such as malic acid, followed by saccharide, nucleobase, and amino acid. Among the 10 types of components, disaccharide has "sweetness" as a characteristic of the flavor. Malic acid and citric acid have "sourness" as a characteristic of the flavor.

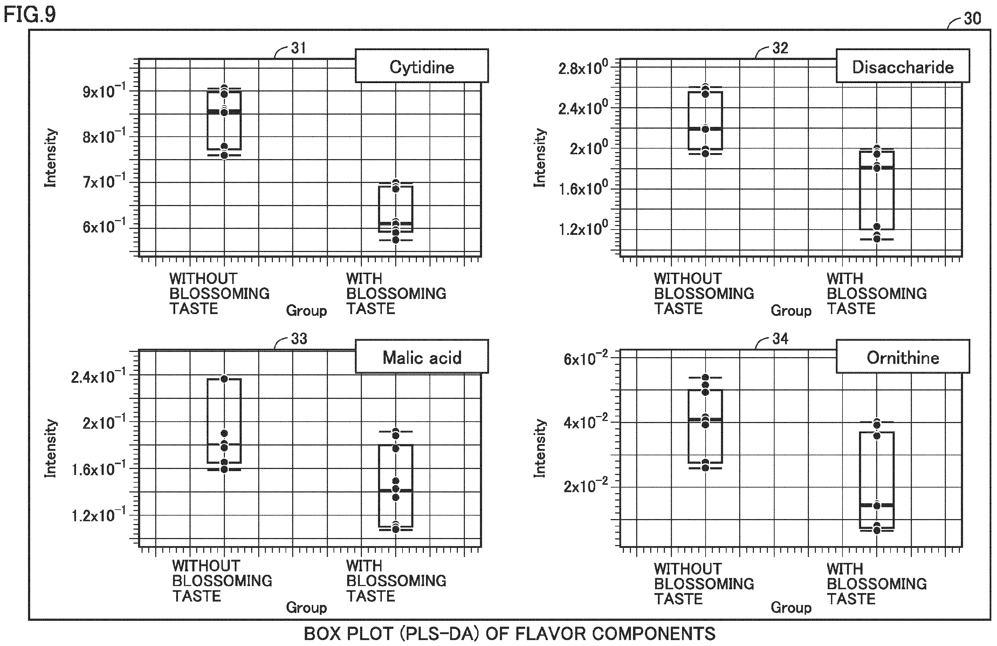

Fig. 9 is a diagram illustrating a difference in the value of the area (the peak area value in a chromatogram) between a sample having blossoming taste and a sample having no blossoming taste, for four types of components among the 10 types of components shown in Fig. 8. In Fig. 9, four graphs 31 to 34 are shown in a frame 30. Graph 31 shows the area value for cytidine, graph 32 shows the area value for disaccharide, graph 33 shows the area value for malic acid, and graph 34 shows the area value for ornithine. Each of graphs 31 to 34 is a box plot.

In each of graphs 31 to 34, the vertical axis represents the peak area value of each component in the chromatogram. "Without blossoming taste" indicates samples (nine samples in total) of three types of sake (Nos. 1, 4, and 8) having no blossoming taste. "With blossoming taste" indicates samples (nine samples in total) of three types of sake (Nos. 2, 5, and 7) having blossoming taste.

It is seen from graphs 31 to 34 that respective contents of all the above four types of components are higher in the samples having no blossoming taste than in the samples having blossoming taste. It is inferred from this fact that it is the balance between components such as organic acid (sourness) and saccharide (sweetness) that influences whether a given type of sake has "blossoming taste" rather than the content of a single component.

### <Display of Marker Component Ratio>

In the present embodiment, the 10 types of components shown in Fig. 8 were selected as components (marker components) that influence whether or not sake has "blossoming taste." Fig. 10 is a diagram illustrating one example of a screen to be provided as information about blossoming taste.

A screen 40 in Fig. 10 includes a graph 41 and a graph 42. Each of graph 41 and graph 42 indicates respective ratios of the above-described 10 types of components in sake. More specifically, for each of the 10 types of components, graph 41 and graph 42 indicate the ratio of the peak area of each component to the sum of the peak areas in the chromatogram of the 10 types of components. It should be noted that graph 41 is the one for No. 7, which is one example of the type having blossoming taste. Graph 42 is the one for No. 1, which is one example of the type having no blossoming taste.

It is easily seen visually from comparison between graph 41 and graph 42 that there is a tendency that the ratio of disaccharide is higher and the ratio of malic acid is lower in the type of sake with blossoming taste than in the type of sake without blossoming taste. It is also easily seen visually that there is a tendency that the ratio of cytidine, which is nucleobase, is lower in the type of sake with blossoming taste than in the type of sake without blossoming taste. From the foregoing, a tendency is derived that the ratio of the sweetness component is higher and the ratio of the sourness component is lower in the type of sake with blossoming taste than in the type of sake without blossoming taste.

Device 100 may present the ratio of each of the 10 types of components as shown in Fig. 10, as an example of information about "blossoming taste" of sake which is a target.

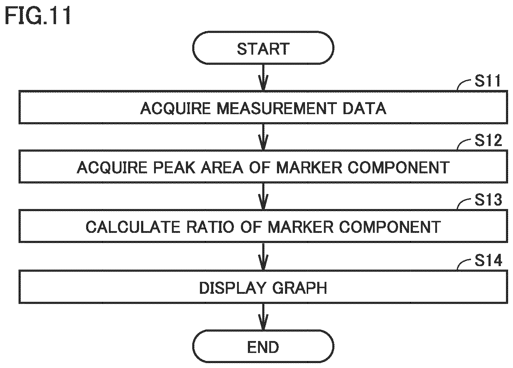

Fig. 11 is a flowchart of one example of a process performed by device 100 to provide information about taste. In one implementation, processor 101 in device 100 executes a given program to thereby perform the process of Fig. 11. In one implementation, the process of Fig. 11 is started in response to input, to mouse 400 and/or keyboard 500, of an instruction to start. In the following, the process of Fig. 11 is described.

In step S11, device 100 acquires (reads) measurement data of a target.

In step 512, device 100 acquires the peak area value of each of marker components from the measurement data acquired in step S11. Instead of the peak area value, the peak height or the component concentration may be acquired. The peak area of each component is acquired by means of the peak picking technique, for example. In the case where device 100 acquires, as the measurement data, the peak area of each component, device 100 reads the peak area of each marker component from the measurement data in step S12.

In step S13, device 100 calculates the ratio of each marker component from the peak area of the marker component acquired in step S12. For example, when the marker components are 10 types of compounds as shown in Fig. 8, the ratio of the peak area of each of the 10 types of components, to the sum of respective peak areas of the 10 types of components is calculated in step S13. The ratio of each component is one example of target ratio information.

In step S14, device 100 uses the ratio of each component calculated in step S13, to produce a graph like graph 41 or graph 42 shown in Fig. 10, and displays the graph on display device 600. Then, device 100 ends the process of Fig. 11.

In step S14 of the process described with reference to Fig. 11, the ratio of the peak area of each of the marker components of a target, to the sum of respective peak areas of the marker components, is displayed as a graph. In this example, the peak area of each component is one example of information about the amount of each component. In this example, it is limited to the marker component that is displayed with its ratio of the peak area. It is thus possible to limit (the number of) components to be considered by a user to determine whether "blossoming taste" is present or not, to the components that are likely to influence whether "blossoming taste" is present or not.

In step S14, device 100 may display the graph produced for the target, together with a graph produced for a type of sake having blossoming taste and a graph produced for a type of sake having no blossoming taste.

Fig. 12 is a diagram illustrating a screen that is displayed in the above-described modification of step S14. In Fig. 12, a screen 45 includes a graph 40X, a graph 41, and a graph 42. Graph 40X represents a graph produced for a target. As shown in Fig. 12, screen 45 displays the graph (graph 40X) for the target, together with the graph (graph 41) produced for a type of sake having blossoming taste and the graph (graph 42) produced for a type of sake having no blossoming taste. A user who watches screen 45 can determine whether or not the target has blossoming taste, based on, for example, whether the ratio in graph 40X is close to the ratio in the type of sake having blossoming taste, or the ratio in the type of sake having no blossoming taste.

### <Generation of Learned Model>

Device 100 uses teacher data 220 to perform a learning process on estimation model 230. In a data set constituting teacher data 220, each piece of data includes information for each sample. More specifically, each piece of data includes, for each sample, information indicating the peak area in a chromatogram of each of the marker components (the above-described 10 types of components) and a determination result (blossoming taste is present or absent) applied to the sample of interest. The peak area is one example of information about the amount of each component in the sample.

Thus, when the peak area of each of the marker components of a target is input to learned estimation model 230, this model outputs a result of determination as to whether or not the target has blossoming taste, by using the peak area of each of the marker components. In device 100, when the peak area of each of the marker components of the target is input to estimation model 230, this model may output respective probabilities of two types of events (the probability that the target has blossoming taste, and the probability that the target does not have blossoming taste), and determination unit 243 may identify an event having the highest probability as the result of determination, to acquire the result of determination.

### <Determination>

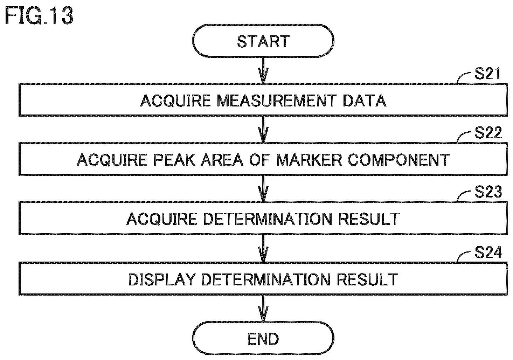

Fig. 13 is a flowchart of another example of the process performed by device 100 to provide information about taste. In one implementation, processor 101 in device 100 executes a given program to thereby perform the process of Fig. 13. In one implementation, the process of Fig. 13 is started in response to input, to mouse 400 and/or keyboard 500, of an instruction to start. In the following, the process of Fig. 13 is described.

In step S21, device 100 acquires (reads) measurement data of a target.

In step S22, device 100 acquires the peak area value of each of marker components from the measurement data acquired in step S21. Instead of the peak area value, the peak height or the component concentration may be acquired. The peak area of each component is acquired by means of the peak picking technique, for example. In the case where device 100 acquires, as the measurement data, the peak area of each component, device 100 reads the peak area of each marker component from the measurement data in step S22.

In step S23, device 100 inputs the peak area of each of the marker components acquired in step S22 to estimation model 230, to thereby acquire a determination result.

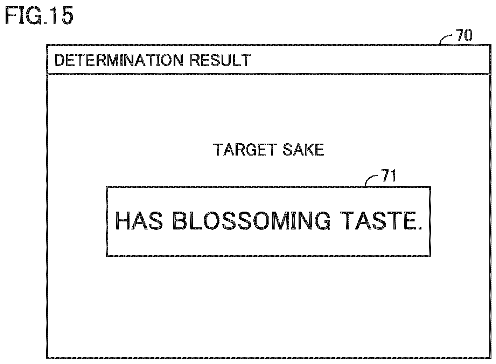

In step S24, device 100 displays the determination result acquired in step S23 on display device 600. One example of a screen for displaying the determination result is described later herein with reference to Fig. 15. Then, device 100 ends the process of Fig. 13.

Device 100 may perform the process of Fig. 13 in parallel with the process of Fig. 11 or continuously with the process of Fig. 11. Device 100 may display the determination result in step S24, together with the graph of step S14 on display device 600.

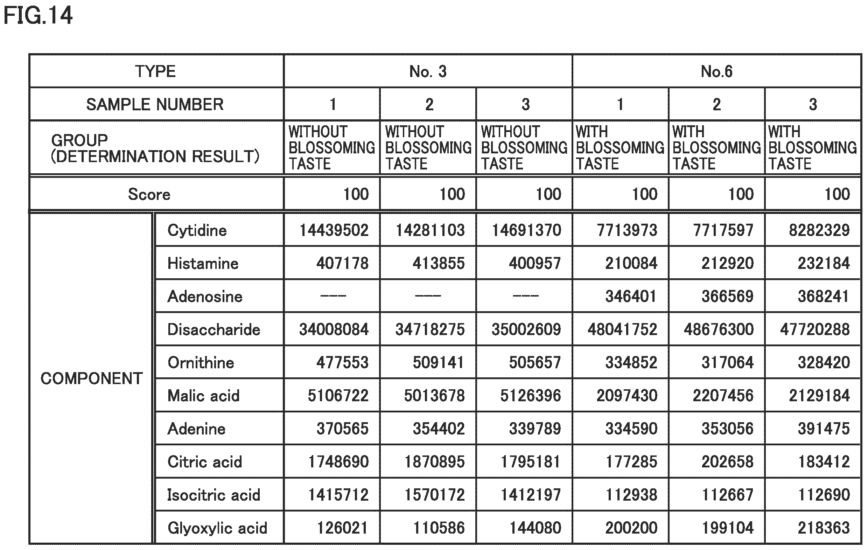

Fig. 14 is a diagram illustrating a specific example of respective peak areas of marker components and a determination result, for two types of targets.

Fig. 14 indicates the type of sake, the sample number, the determination result, the score, and the components.

More specifically, Fig. 14 indicates two types (No. 3 and No. 6 in Fig. 2) as types of sake. In the example of Fig. 14, the targets are therefore the sake of No. 3 and the sake of No. 6 in Fig. 2.

The sample number identifies each of two or more samples produced from a target. In the example of Fig. 14, three samples were produced from each type of sake. Thus, Fig. 14 shows sample numbers 1 to 3 for each type.

The determination result is a result of determination that is output from device 100 for each of the sake of No. 3 and the sake of No. 6. The determination result for each of the three samples of the sake of No. 3 is "without blossoming taste" (having no blossoming taste). The determination result for each of the three samples of the sake of No. 6 is "with blossoming taste" (having blossoming taste).

The score represents a likelihood of the determination result for each sample. The determination result indicates which of the two types of groups (with blossoming taste, and without blossoming taste) each sample belongs to. The score in Fig. 14 indicates that the higher the value of the score, the higher the probability that each sample belongs to the group that is derived as a determination result.

The component indicates the value of the peak area of each marker component that is input to estimation model 230 for acquiring the determination result.

As shown in Fig. 14, the same determination result (without blossoming taste) is derived for all three samples of the sake of No. 3. Moreover, the same determination result (with blossoming taste) is derived for all three samples of the sake of No. 6. Thus, in the case of the determination using learned estimation model 230, a determination result having a certain degree of reliability can be derived for a type of sake evaluated differently by a plurality of assessors. With this determination method, it is possible to determine whether a taste is present or not, in the case where this taste is difficult to identify by only the amount (peak area) of a specific component, and whether or not the taste is present varies depending on the ratio of each of a plurality of different types of components to the total amount of the components. The ratio may be a ratio to 100 defined as the total amount of a plurality of different types of specific components.

Fig. 15 is a diagram illustrating one example of a screen for displaying a determination result. In a field 71 of screen 70 in Fig. 15, a character string "has blossoming taste" which is an example of the determination result is shown. The manner in which the determination result is output is not limited to display of a character string, but may be display of a picture, output of sound, or a combination of them. Thus, such a taste that may be evaluated differently by sensory analysis assessors can be evaluated objectively. In other words, information that supplements a term for expressing a taste by sensory analysis assessors can be provided.

### <Brief Summary>

In the present embodiment described above, information about a specific taste (taste information) of a target is output. The graph displayed in step S14 (Fig. 11) is one example of the taste information. The determination result displayed in step S24 (Fig. 13) is another example of the taste information. Learned estimation model 230 is used to derive the determination result. In this sense, the information (model and parameter) constituting learned estimation model 230 is one example of criterion ratio information, regarding the ratio of the marker component.

In the present embodiment, the taste information is generated by using the amount of a component in a target. Thus, the taste information is provided as objective information for evaluation of a taste. Moreover, the taste information is generated by using the amount (peak area) of each of a plurality of components (marker components), rather than the amount of a single component. Accordingly, the taste information is provided as highly accurate information.

In the present disclosure, "blossoming taste" is one example of specific tastes. The taste to be assessed in the present disclosure is not limited to "blossoming taste."

Another example of specific tastes is "sweetness." In the case of a food having a high content of saccharides as well as a high content of a bitterness or sourness component, a person may be hindered from sensing the sweetness. Thus, according to the present disclosure, the taste information about the taste "sweetness" can be provided by preparing marker components including a component having sweetness as well as a component having bitterness and/or sourness, as characteristics of the flavor, so that the taste information about the taste "sweetness" can be provided.

Another example of specific tastes is "robustness" ("koku" in Japanese). A person may feel "robustness" of a food, in the case where the food has respective contents of a plurality of components that are balanced appropriately, rather than an appropriate content of a single component. Specifically, in the present disclosure, the marker components include a plurality of components contributing to "robustness" so that the taste information about the taste "robustness" can be provided.

Still another example of specific tastes is "savoriness" ("umami" in Japanese). In a food, the balance between and/or the total amount of a plurality of savoriness components (glutamic acid, inosinic acid, and the like) influences the degree to which a person feels savoriness. Specifically, in the present disclosure, the marker components include a plurality of savoriness components, so that the taste information about the taste "savoriness" can be provided.

### [Embodiment 2]

In Embodiment 2, as in Embodiment 1, "blossoming taste" of sake is adopted as one example of specific tastes. While Embodiment 1 focuses on flavor components of sake, Embodiment 2 focuses on aroma components of sake.

### <Selection of Marker Components>

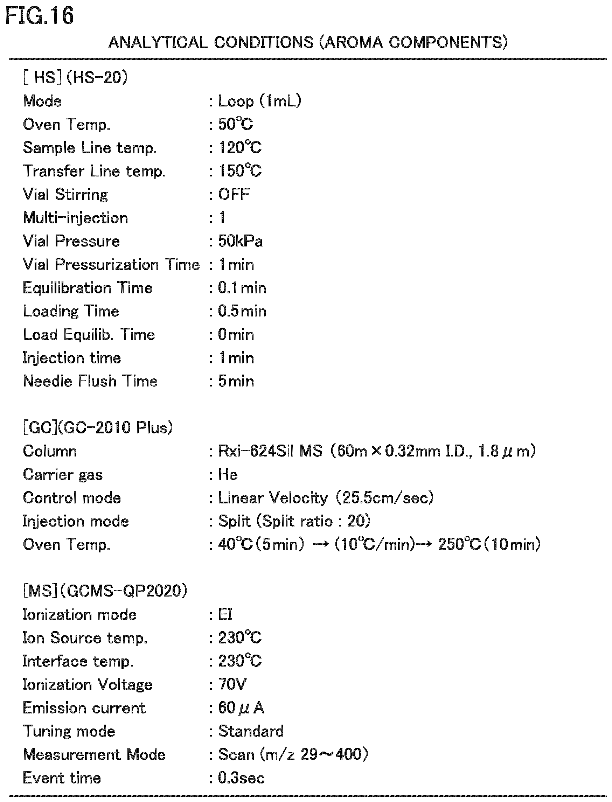

In Embodiment 2, aroma components were identified in eight types of sake (Fig. 2) that are the same as those used in Embodiment 1. Fig. 16 is a diagram illustrating analytical conditions for a gas chromatograph (GC) and a mass spectrometer (MS) for identifying aroma components.

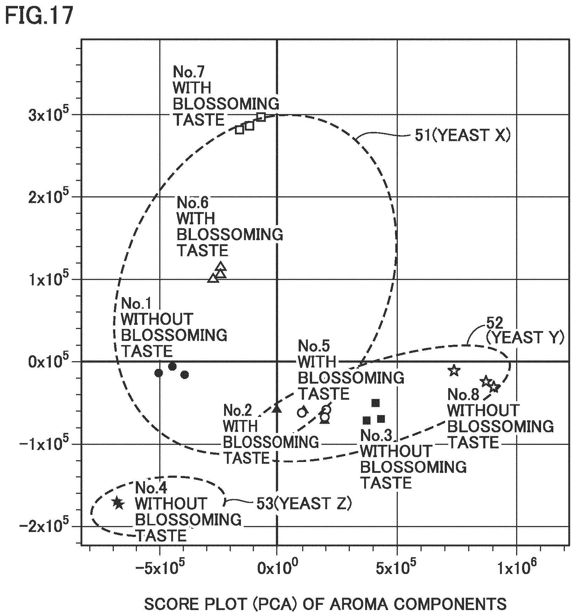

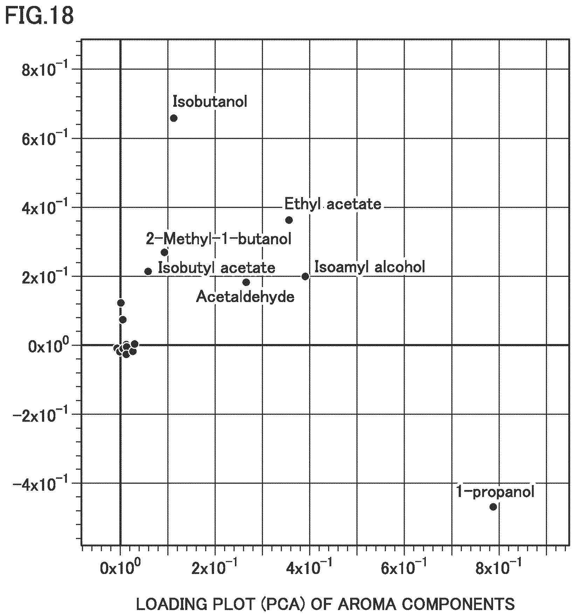

Fig. 17 is a diagram illustrating a score plot produced as a result of principal component analysis on the results of identification of aroma components. Fig. 18 is a diagram illustrating a loading plot produced as a result of principal component analysis on the results of identification of aroma components.

In Fig. 17, regions 51, 52, and 53 each represent a type of yeast (see Fig. 2) used for brewing each type of sake. Region 51 represents a region in which points corresponding to the types of sake (Nos. 1 and 5 to 7) for which yeast X is used are located. Region 52 represents a region in which points corresponding to the types of sake (Nos. 2, 3, and 8) for which yeast Y is used are located. Region 53 represents a region in which points corresponding to the type of sake (No. 4) for which yeast Z is used are located.

In Fig. 18, the names of compounds of principal components are added. The added names of compounds include Isobutanol, Ethyl acetate, 2-Methyl-1-butanol, Isoamyl acetate, Isoamyl alcohol, Acetaldehyde, and 1-propanol.

It is seen from Figs. 17 and 18 that sake may be classified by the type of yeast used for sake, based on principal component analysis, like the one for the flavor components. Yeast X is characterized by various esters and higher alcohols (ethyl acetate, isobutanol, and the like), and yeast Y is characterized by 1-propanol. However, like the flavor components, it is difficult to derive a condition(s) for distinguishing the region where the points "with blossoming taste" are located, from the region where the points "without blossoming taste" are located on the score plot for the aroma components.

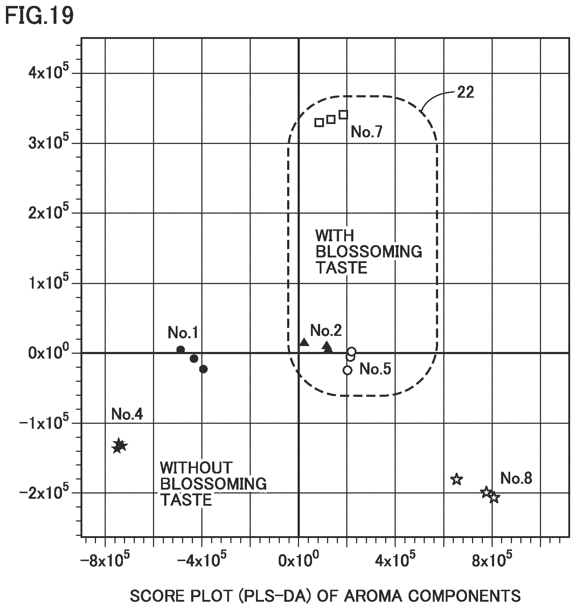

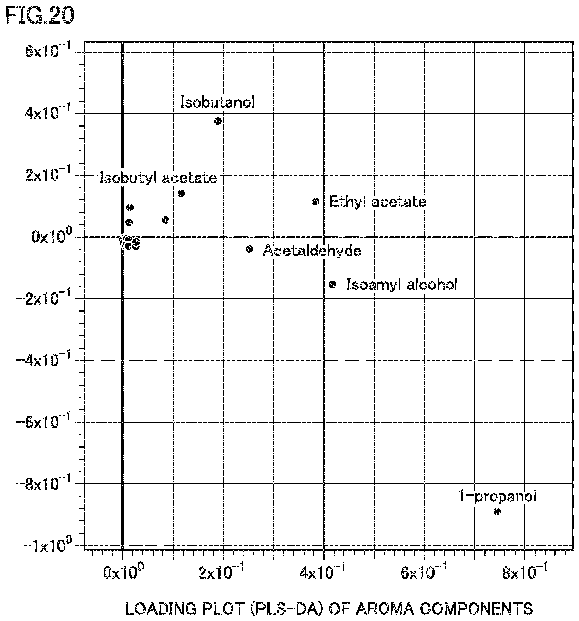

Fig. 19 is a diagram illustrating a score plot produced as a result of partial least squares-discriminant analysis on the results of identification of aroma components. Fig. 20 is a diagram illustrating a loading plot produced as a result of partial least squares-discriminant analysis on the results of identification of aroma components. In Figs. 19 and 20, the types of sake for which a plurality of assessors disagree about evaluation (No. 3 and No. 6 to which the value "R" of "evaluation consistency" is assigned in Fig. 2) are not analyzed. In Fig. 20, Isobutyl acetate is newly added.

It is seen from the score plot illustrated in Fig. 19 that the points for the types of sake "with blossoming taste" (Nos. 2, 5, and 7) are located near the first quadrant and, it is seen from the loading plot that the types classified in a region near the first quadrant in the score plot are characterized by isobutanol, isobutyl acetate, and ethyl acetate.

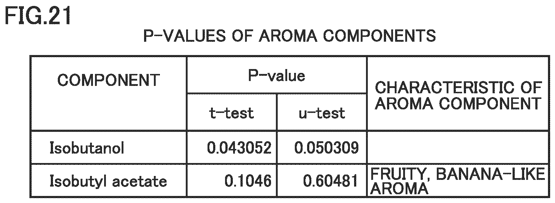

Fig. 21 is a diagram illustrating test statistics of the partial least squares-discriminant analysis described with reference to Figs. 19 and 20. Fig. 21 illustrates respective test statistics (p-values) for the two types of components (isobutanol and isobutyl acetate). These two types of components are contained in No. 1 to No. 8.

In Fig. 21, statistics for t-test and statistics for u-test are shown as test statistics. In the present embodiment, the two types of components shown in Fig. 21 are selected as marker components. Device 100 stores, in memory 200, information for identifying the selected components in association with "blossoming taste." The criterion for this selection is that respective values of the statistic for the t-test and the statistic for the u-test are both 0.2 or less. Isobutyl acetate, which is one of the components, has a fruity, banana-like aroma (a fruit-like aroma of refined sake produced by the ginjo method that uses rice polished well to remove most of bran and ferments the mash slowly at a low temperature), as a characteristic.

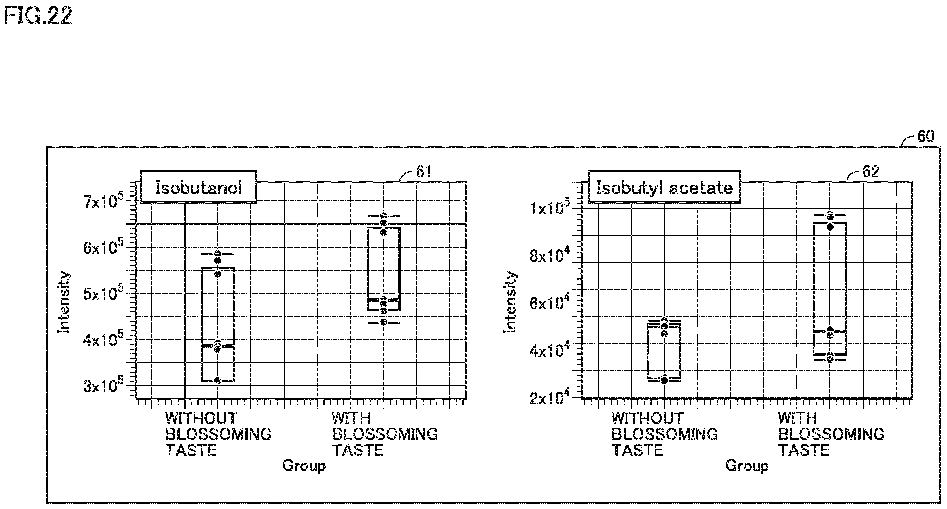

Fig. 22 is a diagram illustrating a difference in the content between a sample having blossoming taste and a sample having no blossoming taste, for the two types of components shown in Fig. 21. In Fig. 22, two graphs 61 and 62 are shown in a frame 60. Graph 61 shows the ratio of isobutanol and graph 62 shows the ratio of isobutyl acetate. Each of graphs 61 and 62 is a box plot. As in Fig. 9, the vertical axis represents the peak area value of each component in the chromatogram. "Without blossoming taste" indicates samples (nine samples in total) of three types of sake (Nos. 1, 4, and 8) having no blossoming taste. "With blossoming taste" indicates samples (nine samples in total) of three types of sake (Nos. 2, 5, and 7) having blossoming taste.

It is seen from graphs 61 and 62 that respective contents of both of the above two types of components are higher in the samples having blossoming taste than in the samples having no blossoming taste. It is inferred from this fact that it is the amount of each of the two types of components that influences whether a given type of sake has "blossoming taste" rather than the content of a single component.

### <Generation of Learned Model>

Device 100 uses teacher data 220 to perform a learning process on estimation model 230. In a data set constituting teacher data 220, each piece of data includes information for each sample. More specifically, each piece of data includes, for each sample, information indicating the peak area in a chromatogram of each of the marker components (the above-described two types of components) and a determination result (blossoming taste is present or absent) applied to the sample of interest. The peak area is one example of information about the amount of each component in the sample. Thus, when the peak area of each of the marker components of a target is input to learned estimation model 230, this model outputs a result of determination as to whether or not the target has blossoming taste, by using the peak area of each of the marker components.

### <Determination>

In the present embodiment, like Embodiment 1, device 100 may also provide information about taste, in accordance with the flowchart illustrated in Fig. 13.

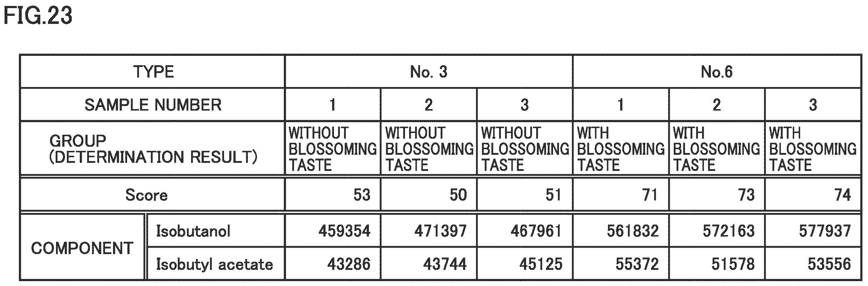

Fig. 23 is a diagram illustrating a specific example of respective peak areas of marker components and a determination result, for two types of targets. Similarly to Fig. 14, Fig. 23 indicates the type of sake, the sample number, the determination result, the score, and the components, for two types (No. 3 and No. 6 in Fig. 2). In Fig. 23, the two types of aroma components as described above are shown each as a type of component.

As shown in Fig. 23, the same determination result (without blossoming taste) is derived for all three samples of the sake of No. 3. Moreover, the same determination result (with blossoming taste) is derived for all three samples of the sake of No. 6. Thus, in the case of the determination using learned estimation model 230, a determination result having a certain degree of reliability can be derived for a type of sake evaluated differently by a plurality of assessors.

### [Embodiment 3]

In Embodiment 3, as in Embodiment 1, "blossoming taste" of sake is adopted as one example of specific tastes. In Embodiment 3, a result of determination as to whether or not a target has a specific taste is acquired, in accordance with a predetermined criterion.

### <Device Configuration>

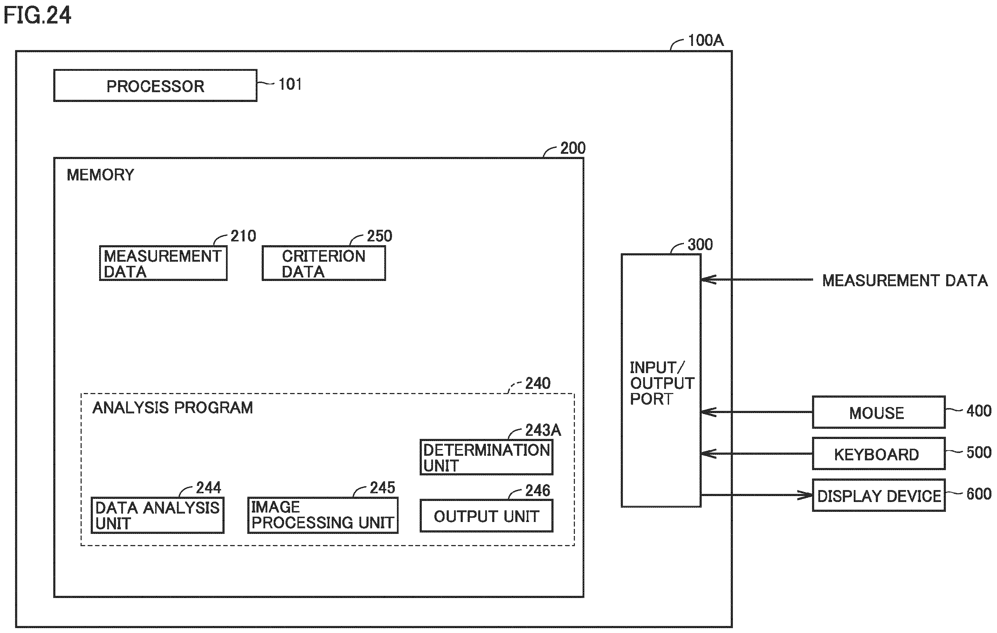

Fig. 24 is a block diagram illustrating a configuration of a device 100A for providing taste information. In device 100A illustrated in Fig. 24, criterion data 250 is stored in a memory 200. Criterion data 250 is one example of criterion ratio information, and represents a criterion for sake, which is a target, to have (or not to have) "blossoming taste."

In device 100A illustrated in Fig. 24, functions of an analysis program 240 include a determination unit 243A. Determination unit 243A derives a determination result of whether or not a target has a specific taste, based on measurement data 210 and criterion data 250.

In memory 200, information for identifying marker components selected as described in connection with Embodiment 1 is stored in association with "blossoming taste."

### <Determination>

Criterion data 250 defines a criterion for determining that a target has "blossoming taste."

One example of the criterion defines the ratio of disaccharide and the ratio of malic acid, to the sum of the 10 types of marker components selected using the result of the partial least squares-discriminant analysis in Embodiment 1, in terms of the peak area in the chromatogram. More specifically, the ratio of disaccharide is 66 to 71% and the ratio of malic acid is 4.0 to 5.0%.

In this case, device 100A calculates the sum of respective peak areas of the 10 types of marker components, from the measurement data of the target, and calculates the ratio of the peak area of disaccharide to the sum and the ratio of the peak area of malic acid to the sum. When both of the two calculated ratios fall within respective ranges defined by the above-described criterion, device 100A outputs a determination result that the target has "blossoming taste." In contrast, when at least one of the two calculated ratios is outside the respective range defined by the criterion, device 100A outputs a determination result that the target does not have "blossoming taste."

Criterion data 250 may define a criterion for determining that a target does not have "blossoming taste."

Another example of the criterion is a criterion that the ratio of disaccharide is 62 to 63% and the ratio of malic acid is 5.5 to 8.0%, to the sum of the 10 types of marker components. When both of the two calculated ratios fall within respective ranges defined by the criterion, device 100A outputs a determination result that the target does not have "blossoming taste." In contrast, when at least one of the two calculated ratios is outside the respective range defined by the criterion, device 100A outputs a determination result that the target has "blossoming taste."

The names and the numerical values for the components described above are given merely as one example. When the technique according to the present disclosure is implemented, the names and/or the numerical values for the components may be changed appropriately.

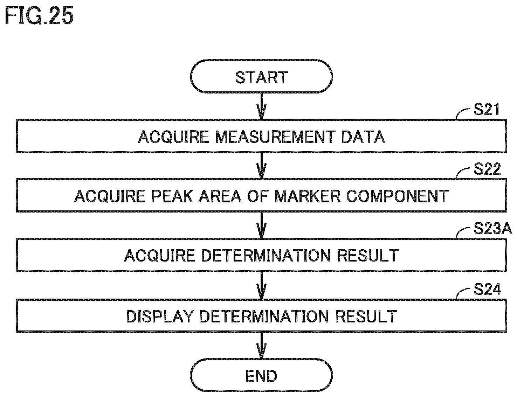

Fig. 25 is a flowchart of one example of a process performed by device 100A. The process of Fig. 25 includes step S23A instead of step S23, as compared with the process illustrated in Fig. 13.

More specifically, device 100A acquires (reads) measurement data of a target in step S21, and acquires the peak area value of each of marker components in step S22. Instead of the peak area value, the peak height or the component concentration may be acquired.

In step S23A, device 100A acquires a determination result, by using respective peak areas of the marker components acquired in step S22 and the criterion stored in criterion data 250.

In step S24, device 100A displays the determination result acquired in step S23A on a display device 600. The determination result is displayed as a screen 70 of Fig. 15, for example. Then, device 100A ends the process of Fig. 25.

### [Embodiment 4]

Device 100 or device 100A can provide the information that is output in each of Embodiments 1 to 3, for each of a plurality of types of tastes. Device 100 or device 100A may provide information about each of a plurality of types of tastes to a user. Device 100 or device 100A may receive selection, from a plurality of types of tastes, of a type of taste about which information is to be provided. Device 100 or device 100A uses the information on the selected type to provide taste information about the selected type.

In one implementation, memory 200 of device 100 stores information for identifying marker components specified for each of a plurality of types of tastes, as well as criterion ratio information for the marker components. For estimation model 230, a learning process has been performed for each of the plurality of types of tastes. That is, memory 200 stores, as parameters of estimation model 230, parameter sets resultant from the learning process for each of the plurality of types of tastes.

In one implementation, memory 200 of device 100A stores information for identifying marker components specified for each of a plurality of types of tastes, as well as criterion ratio information for the marker components. Memory 200 stores criterion ratio information for each of the plurality of types of tastes, as criterion data 250.

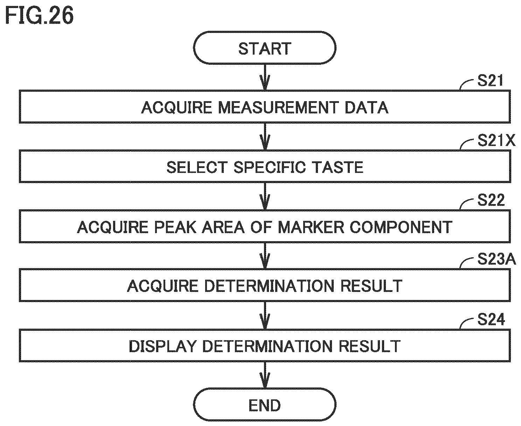

Fig. 26 is a flowchart of one example of a process performed to provide information about taste in Embodiment 4. As a process in Embodiment 4, a modification of the process (Fig. 25) described in connection with Embodiment 3 is described.

The process of Fig. 26 further includes step S21X as compared with the process of Fig. 25. More specifically, after acquiring measurement data of a target in step S21, device 100A proceeds to step S21X. In step S21X, device 100A receives, from a user, selection of a type of taste of interest from a plurality of types of tastes. Then, criterion ratio information about the received type of taste is selected from criterion data 250. Thereafter, device 100A performs control in step S22 and subsequent steps, for the taste of the selected type.

### [Aspects]

It is to be understood by those skilled in the art that a plurality of exemplary embodiments described above are specific examples of the following aspects.

(Clause 1) A taste information providing method according to one aspect is a method for providing taste information about a specific taste, and the method may include: acquiring target ratio information about respective ratios of two or more types of components in a target, the two or more types of components being associated with the specific taste; deriving a determination result for the specific taste of the target, based on the target ratio information and criterion ratio information about respective ratios of the two or more types of components; and outputting the determination result.

With the taste information providing method according to Clause 1, objective and highly accurate information is provided for taste evaluation accomplished by combining a plurality of types of components.

(Clause 2) In the taste information providing method according to Clause 1, the two or more types of components may include a first component and a second component, and the criterion ratio information may define a criterion for a ratio of an amount of the first component to a sum of respective amounts of the two or more types of components, and a ratio of an amount of the second component to the sum of respective amounts of the two or more types of components.

With the taste information providing method according to Clause 2, more specific information is provided regarding evaluation of the taste determined by respective specific ratios of the first component and the second component.

(Clause 3) In the taste information providing method according to Clause 2, the first component may be disaccharide, and the second component may be malic acid.

With the taste information providing method according to Clause 3, more specific information is provided regarding evaluation of the taste including disaccharide and malic acid.

(Clause 4) In the taste information providing method according to any one of Clauses 1 to 3, deriving the determination result may include deriving a determination result as to whether the specific taste is present.

With the taste information providing method according to Clause 4, specific information about the determination result as to whether or not the specific taste is present can be provided.

(Clause 5) The taste information providing method according to any one of Clauses 1 to 4 may further include selecting the specific taste from a plurality of tastes.

With the taste information providing method according to Clause 5, a user can selectively acquire only information about a type of taste that the user wants to obtain, from a plurality of types of taste.

(Clause 6) The taste information providing method according to any one of Clauses 1 to 5 may include acquiring respective component amounts of the two or more types of components in the target, and acquiring the target ratio information may include acquiring the target ratio information based on the component amounts.

With the taste information providing method according to Clause 6, the target ratio information is calculated to more accurately represent the ratio in the target.

(Clause 7) The taste information providing method according to Clause 2 may further include: calculating a ratio of an amount of each component to a sum of respective amounts of the two or more types of components, based on information about an amount of each component of the two or more types of components; and outputting the ratio of the amount of each component.

With the taste information providing method according to Clause 7, the basis for the determination result can be provided by displaying respective ratios of components determining a specific taste.

(Clause 8) A taste information providing method according to one aspect is a method for providing taste information about a specific taste, and the method may include: acquiring information about an amount of each component of two or more types of components in a target, the two or more types of components being associated with the specific taste; calculating a ratio of the amount of each component to a sum of respective amounts of the two or more types of components, based on the information about the amount of each component of the two or more types of components; and outputting the ratio of the amount of each component.

With the taste information providing method according to Clause 8, objective and highly accurate information is provided for evaluation of taste determined by a combination of respective ratios of components that cannot be evaluated based on only the amount of a characteristic component of the target.

(Clause 9) In the taste information providing method according to Clause 7 or 8, outputting the ratio of the amount of each component includes outputting the ratio of each component in a sample having the specific taste and the ratio of each component in a sample without the specific taste.

With the taste information providing method according to Clause 9, a user is given more materials for determination of a specific taste of a target.

(Clause 10) In the taste information providing method according to any one of Clauses 1 to 9, the two or more types of components may be selected, based on a test statistic calculated for each component of one or more samples having the specific taste and one or more samples without the specific taste for analyzing, by using partial least squares-discriminant analysis, a difference between the one or more samples having the specific taste and the one or more samples without the specific taste.

With the taste information providing method according to Clause 10, components characterizing a specific taste are selected as the two or more types of components.

(Clause 11) In the taste information providing method according to any one of Clauses 1 to 8, the specific taste may be blossoming taste of sake.

With the taste information providing method according to Clause 11, objective and highly accurate information is provided about "blossoming taste" referred to for sake.

(Clause 12) In the taste information providing method according to Clause 11, the two or more types of components may include at least some in a group consisting of cytidine, histamine, adenosine, disaccharide, ornithine, adenine, malic acid, citric acid, isocitric acid, and glyoxylic acid.

With the taste information providing method according to Clause 12, more accurate information about "blossoming taste" is provided.

(Clause 13) A program according to one aspect is a program for providing taste information, and the program may be executed by a processor of a computer to cause the computer to perform the taste information providing method according to any one of Clauses 1 to 12.

With the program according to Clause 11, objective and highly accurate information regarding taste evaluation is provided by the program.

It should be construed that the embodiments disclosed herein are given by way of illustration in all respects, not by way of limitation. It is intended that the scope of the present disclosure is defined by claims, not by the above description of the embodiments, and encompasses all modifications and variations equivalent in meaning and scope to the claims. It is also intended that each technique in the embodiments may be implemented singly or in combination to a possible extent with another technique in the embodiments as required.

### REFERENCE SIGNS LIST

11, 12, 13, 51, 52, 53 region; 31, 32, 33, 34, 40X, 41, 42, 61, 62 graph; 40, 45, 70 screen; 71 field; 100, 100A device; 101 processor; 200 memory; 210 measurement data; 220 teacher data; 230 estimation model; 240 analysis program; 241 data generation unit; 242 model generation unit; 243, 243A determination unit; 244 data analysis unit; 245 data processing unit; 246 output unit; 250 criterion data; 300 input/output port; 400 mouse; 500 keyboard; 600 display device.

## Claims

1. A taste information providing method for providing taste information about a specific taste, the method comprising:
acquiring target ratio information about respective ratios of two or more types of components in a target, the two or more types of components being associated with the specific taste;
deriving a determination result for the specific taste of the target, based on the target ratio information and criterion ratio information about respective ratios of the two or more types of components; and
outputting the determination result.

2. The taste information providing method according to claim 1, wherein
the two or more types of components include a first component and a second component, and
the criterion ratio information defines a criterion for a ratio of an amount of the first component to a sum of respective amounts of the two or more types of components, and a ratio of an amount of the second component to the sum of respective amounts of the two or more types of components.

3. The taste information providing method according to claim 2, wherein the first component is disaccharide, and the second component is malic acid.

4. The taste information providing method according to claim 1, wherein deriving the determination result includes deriving a determination result as to whether the specific taste is present.

5. The taste information providing method according to claim 1, further comprising selecting the specific taste from a plurality of tastes.

6. The taste information providing method according to claim 1, further comprising acquiring respective component amounts of the two or more types of components in the target, wherein
acquiring the target ratio information includes acquiring the target ratio information based on the component amounts.

7. The taste information providing method according to claim 2, further comprising:
calculating a ratio of an amount of each component to a sum of respective amounts of the two or more types of components, based on information about an amount of each component of the two or more types of components; and
outputting the ratio of the amount of each component.

8. A taste information providing method for providing taste information about a specific taste, the method comprising:
acquiring information about an amount of each component of two or more types of components in a target,
the two or more types of components being associated with the specific taste;
calculating a ratio of the amount of each component to a sum of respective amounts of the two or more types of components, based on the information about the amount of each component of the two or more types of components; and
outputting the ratio of the amount of each component.

9. The taste information providing method according to claim 7, wherein outputting the ratio of the amount of each component includes outputting the ratio of each component in a sample having the specific taste and the ratio of each component in a sample without the specific taste.

10. The taste information providing method according to claim 1, wherein the two or more types of components are selected, based on a test statistic calculated for each component of one or more samples having the specific taste and one or more samples without the specific taste for analyzing, by using partial least squares-discriminant analysis, a difference between the one or more samples having the specific taste and the one or more samples without the specific taste.

11. The taste information providing method according to claim 1, wherein the specific taste includes blossoming taste of sake.

12. The taste information providing method according to claim 11, wherein the two or more types of components include at least some in a group consisting of cytidine, histamine, adenosine, disaccharide, ornithine, adenine, malic acid, citric acid, isocitric acid, and glyoxylic acid.

13. A program for providing taste information, the program being executed by a processor of a computer to cause the computer to perform the taste information providing method according to claim 1.
